# EUROPÄISCHE PATENTSCHRIFT

(11) **EP 1 943 201 B1**
(45) Veröffentlichungstag und Bekanntmachung des Hinweises auf die Patenterteilung: **18.09.2013**
(21) Anmeldenummer: 06807664.5
(22) Anmeldetag: 30.10.2006
(51) Int. Cl.: C07C 2/84, C07C 15/00

(54) **VERFAHREN ZUR SYNTHESE VON AROMATISCHEN KOHLENWASSERSTOFFEN AUS C1-C4-ALKANEN UND VERWERTUNG VON C1-C4-ALKAN-HALTIGEM PRODUKTSTROM**
METHOD FOR THE SYNTHESIS OF AROMATIC HYDROCARBONS FROM C1-C4 ALKANES, AND UTILIZATION OF A C1-C4 ALKANE-CONTAINING PRODUCT FLOW
PROCEDE DE SYNTHESE D'HYDROCARBURES AROMATIQUES A PARTIR DE C1-C4-ALCANES ET UTILISATION D'UN FLUX DE PRODUIT CONTENANT DES C1-C4-ALCANES

(30) Priorität: 28.10.2005 DE 102005052094; 27.04.2006 EP 06113231
(43) Veröffentlichungstag der Anmeldung: 16.07.2008
(73) Patentinhaber: BASF SE, 67056 Ludwigshafen (DE)
(72) Erfinder: KIESSLICH, Frank, 63128 Dietzenbach (DE); CRONE, Sven, 67117 Limburgerhof (DE); MACHHAMMER, Otto, 68163 Mannheim (DE); VAN LAAR, Frederik, Dubai (AE); SCHWAB, Ekkehard, 67434 Neustadt (DE); SCHINDLER, Götz-Peter, 67071 Ludwigsfafen (DE)
(86) Internationale Anmeldenummer: PCT/EP2006/067938
(87) Internationale Veröffentlichungsnummer: WO 2007/048853

(56) Entgegenhaltungen:
- EP-A1- 0 269 297
- US-A- 3 827 867
- US-A1- 2003 144 565
- QI S ET AL: "Methane aromatization using Mo-based catalysts prepared by microwave heating" CATALYSIS TODAY, ELSEVIER, Bd. 98, Nr. 4, 14. Dezember 2004 (2004-12-14), Seiten 639-645, XP004657597 ISSN: 0920-5861 in der Anmeldung erwähnt

## Beschreibung

Die vorliegende Erfindung betrifft ein Verfahren zur Herstellung von aromatischen Kohlenwasserstoffen, wie Benzol, Toluol, Ethylbenzol, Styrol, Xylol, Naphthalin oder Gemischen hiervon, aus C₁-C₄-Alkanen, und die Verwertung von nicht umgesetzten C₁-C₄-Alkanen, in einem weiteren C₁-C₄-Alkan-verbrauchenden, insbesondere Methan-verbrauchenden, Verfahren.

Aromatische Kohlenwasserstoffe, wie Benzol, Toluol, Ethylbenzol, Styrol, Xylol und Naphthalin, stellen bedeutende Zwischenprodukte in der chemischen Industrie dar, deren Bedarf nach wie vor steigt. In der Regel werden sie durch katalytische Reformierung aus Naphtha gewonnen, das seinerseits aus Erdöl erhalten wird. Neuere Untersuchungen zeigen, dass die weltweiten Erdölvorräte im Vergleich zu den Erdgasvorräten limitierter sind. Daher ist es erstrebenswert aromatische Kohlenwasserstoffe aus Edukten, die aus Erdgas gewonnen werden können, herzustellen. Die Hauptkomponente von Erdgas stellt Methan dar (typische Zusammensetzung von Erdgas: 75 bis 99 mol% Methan, 0,01 bis 15 mol% Ethan, 0,01 bis 10 mol% Propan, bis zu 0,06 mol% Butan und höhere Kohlenwasserstoffe, bis 0,30 mol% Kohlendioxid, bis 0,30 mol% Schwefelwasserstoff, bis zu 0,15 mol% Stickstoff, bis zu 0,05 mol% Helium).

Anderson et al. berichten in Applied Catalysis 19, 141 (1985), dass es möglich ist, Methan mit Stickoxid (N₂O) in Gegenwart des Katalysators H-ZSM-5 zu Benzol umzusetzen. In der Folgezeit stellten Claridge et al. (Applied Catalysis, A: General 89, 103 (1992) fest, dass Methan mit Sauerstoff, insbesondere in Gegenwart von Chloridpromotiertem Mangan(IV)oxid, u.a. zu Benzol umgesetzt werden kann.

Weiterhin beschreiben Wang et al. in Catalytic Letters 21, 35 (1993) ein Verfahren zur Dehydrierung und Aromatisierung von Methan unter nicht-oxidtiven Bedingungen. Mit den hierbei verwendeten ZSM-5 Zeolith-Katalysatoren, die mit Molybdän bzw. Zink modifiziert werden, wird ein Methan-Umsatz von max. 7,2 % erreicht. Weiterhin berichten Qi et al. in Catalysis Today 98, 639 (2004), dass der Methan-Umsatz und die Benzol-Selektivität an dem Mo/ZSM-5 Zeolith-Katalysator u.a. durch Zusatz von Kupfer als Promotor erhöht werden kann.

EP-A 0 269 297 beschreibt die Aromatisierung von C₂-C₄-Alkanen, die Auftrennung des Produktstroms in Aromaten, einen methanreichen und einen wasserstoffreichen Gasstrom, Umsetzung des methanreichen Gasstroms in Synthesegas und die gemeinsame Reaktion des Synthesegases mit dem wasserstoffreichen Strom gemäß Fischer-Tropsch zu Kohlenwasserstoffen. Die Verwendung eines Eduktstroms, der mindestens 70 mol-% Methan enthält, wird nicht offenbart.

Die bisher beschriebenen Verfahren weisen niedrige Umsätze an Methan auf. Daher wird beispielsweise in US 2003/0144565 vorgeschlagen den Produktstrom, von dem die gebildeten aromatischen Kohlenwasserstoffe abgetrennt wurden, zurückzuführen und so das unumgesetzte Methan weiter zu nutzen. Allerdings senken Rückführungsströme die wirtschaftliche Effizienz, da zum einen weitere verfahrungstechnische Operationen, wie erneute Erwärmung, Verdichtung etc. durchgeführt werden müssen und zudem größere Reaktorvolumina bereitgestellt werden müssen. Außerdem kann es zur Anreicherung von unerwünschten Nebenkomponenten oder gar zu unerwünschten Nebenreaktionen kommen.

Aufgabe der vorliegenden Erfindung ist die Bereitstellung eines Verfahrens zur Herstellung von aromatischen Kohlenwasserstoffen aus C₁-C₄-Alkanen, wobei die eingesetzten C₁-C₄-Alkane, effizient genutzt werden.

Gelöst wird die Aufgabe durch ein Verfahren zur Herstellung von aromatischen Kohlenwasserstoffen aus einem C₁-C₄-Alkan, oder einem Gemisch von C₁-C₄-Alkanen, dadurch gekennzeichnet, dass man
a) einen Eduktstrom A, der ein C₁-C₄-Alkan, oder ein Gemisch von C₁-C₄-Alkanen, enthält, wobei der Eduktstrom A mindestens 70 mol-% Methan enthält, mit einem Katalysator in Kontakt bringt, und einen Teil des C₁-C₄-Alkans, oder einen Teil des Gemisches der C₁-C₄-Alkane, zu aromatischen Kohlenwasserstoff(en), umsetzt;
b) den aus Schritt a) resultierenden Produktstrom B, in einen Leichtsiederstrom C, welcher den Hauptteil des Wasserstoffs und des unumgesetzten C₁-C₄-Alkans, oder des Gemisches von C₁-C₄-Alkanen, enthält, und einen Schwersiederstrom D oder mehrere Schwersiederströme D', welcher oder welche den Hauptteil des gebildeten aromatischen Kohlenwasserstoffes enthält oder enthalten, auftrennt; und
c) den Leichtsiederstrom C einem weiteren C₁-C₄-Alkan-verbrauchenden Verfahren, ausgewählt aus der Gruppe
   i) Verbrennung in einem Gas- und Dampf-Kraftwerk;
   ii) Darstellung von Synthesegas durch Dampf-Reformieren oder partieller Oxidation;
   iii) Umsetzung mit Ammoniak zu Blausäure in Gegenwart von Sauerstoff nach dem Andrussow-Verfahren, oder ohne Sauerstoffzusatz nach dem BMA-Verfahren;
   iv) Umsetzung mit Schwefel zu Schwefelkohlenstoff;
   v) Pyrolyse zu Acetylen im Lichtboden oder nach dem Sachsse-Bartholome-Verfahren; und
   vi) oxidative Kupplung zu Ethylen;
zuführt.

In einer Ausführungsform enthält der Eduktstrom A bevorzugt mindestens 80 mol%, insbesondere mindestens 90 mol% C₁-C₄-Alkan.

Insbesondere kann als Eduktstrom A Gas, das einen Anteil von mindestens 70 mol% Methan, bevorzugt von mindestens 75 mol% Methan enthält, eingesetzt werden. In der Regel enthält der Eduktstrom neben Methan noch Ethan, üblicherweise 0,01 bis 15 mol%, Propan, üblicherweise 0,01 bis 10 mol%, ggf. Butan und höhere Kohlenwasserstoffe, üblicherweise 0 bis 0,06 mol. Der Anteil an aromatischen Kohlenwasserstoffen ist in der Regel unter 2 mol% und vorzugsweise kleiner als 0.5 mol%.

In einer weiteren Ausführungsform kann als Eduktstrom A LPG (liquid petroleum gas) eingesetzt werden.

In einer weiteren Ausführungsform kann als Eduktstrom A LNP (liquified natural gas) eingesetzt werden.

Weiterhin kann der Eduktstrom A Stickstoff, üblicherweise 0 bis zu 0,15 mol%, Schwefelwasserstoff, üblicherweise 0 bis 0,30 mol% und/oder anderen Verunreinigungen, üblicherweise 0 bis 0,30 mol%, enthalten.

In einer weiteren Ausführungsform kann dem Eduktstrom A zusätzlich Wasserstoff, Wasserdampf, Kohlenmonoxid, Kohlendioxid, Stickstoff, ein oder mehrere Edelgase und/oder ein sauerstoffhaltiger Gasstrom zugesetzt werden. Als sauerstoffhaltige Gasströme kommen beispielsweise Luft, angereicherte Luft, Reinsauerstoff in Betracht.

In einer besonderen Ausführungsform wird der Eduktstrom A pur verwendet.

Je nach gewählter Fahrweise und Reaktionsbedingungen kann eine sehr hohe Konzentration oder eine starke Verdünnung der C₁-C₄-Alkane in einem der oben erwähnten Gasströme von großem Vorteil sein. Das Volumenverhältnis zwischen dem Eduktstrom A und dem zugesetzten Gasstrom kann je nach Verfahren innerhalb sehr weiter Grenzen variieren. Typischerweise liegt dieses im Bereich von 1000:1 bis 1:500, bevorzugt 1000:1 bis 1:100, besonders bevorzugt, insbesondere 1000:1 bis 1:50.

Die Zugabe kann dabei in Form eines stetigen Stromes oder einer instationären oder periodischen Weise erfolgen.

In einer speziellen Ausführungsform kann auch die Dosierung einzelner Komponenten in Spuren von nur einigen ppm zum Eduktstrom A erfolgen.

Die dehydrierende Aromatisierung von C₁-C₄-Alkanen gemäß der vorliegenden Erfindung kann unter Zufuhr oder ohne Zufuhr von sauerstoffstoffhaltigen Gasen, an bekannten Katalysatoren, unter, für den Fachmann bekannten Bedingungen, durchgeführt werden.

Als Katalysatoren eignen sich insbesondere Zeolith-haltige Katalysatoren. Diese Zeolithe haben in der Regel einen Porenradius zwischen 5 und 7 Angström. Beispiele sind hierfür ZSM-Zeolithe, wie beispielsweise ZSM-5, ZSM-8, ZSM-11, ZSM-23 und ZSM-35, vorzugsweise ZSM-5, oder MCM-Zeolithe, wie beispielsweise MCM-22. Die Katalysatoren können neben den Zeolithen ein oder mehrere Metalle aus den Gruppen IIA, IIIA,IB, IIB, IIIB, VIB, VIIB und VIIIB enthalten.

Vorzugsweise werden Mo/HZSM-5-Katalysatoren verwendet, welche mit Cu, Co, Fe, Pt, Ru promotiert sein können. Es ist aber auch möglich mit Sr, La oder Ca zu promotieren. Es können aber auch W/HZSM-5, In/HZSM-5, Ga/HZSM-5, Zn/HZSM-5, Re/HZSM-5 eingesetzt werden, aber auch W/HZSM-5, promotiert mit Mn, Zn, Ga, Mo oder Co.

Ebenso vorzugsweise werden W/MCM-22-Katalysatoren verwendet, welche mit Zn, Ga, Co, Mo promotiert sein können. Es kann aber auch Re/HMCM-22 eingesetzt werden.

Ebenso kann Chlorid-promotiertes Mangan(IV)oxid, H-ZSM-5 und Cu-W/HZSM-5 eingesetzt werden oder auch Rh auf einem SiO₂-Träger.

In einer Ausgestaltungsform wird unter Zufuhr von sauerstoffhaltigen Komponenten gearbeitet. Als Oxidationsmittel können dem Fachmann bekannte, für Gasphasen-Reaktionen übliche Oxidationsmittel, wie beispielsweise Sauerstoff, angereicherte Luft oder Luft verwendet werden. Alternativ können je nach Verfügbarkeit unter Umständen auch andere Oxidationsmittel, wie z. B. Stickoxide (NOₓ, N₂O) genutzt werden. Hierbei kann das Oxidationsmittel vor dem Reaktor mit dem Eduktstrom A vereinigt werden oder dieses an einer oder mehrerer Stellen in der Reaktionszone zudosiert werden. Die einmalige Zugabe als auch die Zugabe in mehreren Portionen ist denkbar.

Als spezielle Ausgestaltungsform ergibt sich die autotherme Fahrweise. Unter autothermer Fahrweise versteht man bei endothermen Reaktionen die Erzeugung der Wärme für den Prozess aus dem Reaktionsgemisch selbst heraus. Hierzu wird die endotherme Ziel-Reaktion mit einer zweiten Reaktion thermisch gekoppelt, welche über ihre Exothermie die Wärmebilanz ausgleicht. Wärmezufuhr zur Reaktionszone durch ein externes Heizmedium von außen wird hierdurch vermieden. Wärmeintegration innerhalb des Prozesses kann jedoch nach wie vor genutzt werden.

Die autotherme Fahrweise kann in verschiedenster, für den Fachmann bekannte Art und Weise erfolgen.

Hierbei wird eine zweite Reaktion, welche exotherm verläuft, genutzt, um die Endothermie der dehydrierenden Aromatisierung thermisch auszugleichen. Bevorzugt handelt es sich bei dieser exothermen Reaktion um eine Oxidation. Verschiedene Oxidationsmittel können hierbei genutzt werden. Üblicherweise finden Sauerstoff, sauerstoffhaltige Gemische oder Luft Verwendung.

Im Allgemeinen wird die Menge des dem Reaktionsgasgemisches zugesetzten sauerstoffhaltigen Gasstromes so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die dehydrierende Aromatisierung benötigte Wärmemenge erzeugt wird. Üblicherweise wird ein Verhältnis O:C-Atom (mol/mol) von 1:12 bis 1:1, vorzugsweise 1:10 bis 1:1,5, insbesondere 1:15 bis 1:2 eingesetzt. Als sauerstoffhaltiger Gasstrom wird ein sauerstoffhaltiges Gas, welches Inertgase enthält, beispielsweise Luft, oder mit Sauerstoff angereicherte Luft, oder Sauerstoff, eingesetzt.

Der zur Wärmeerzeugung verbrannte Wasserstoff ist der bei der dehydrierenden Aromatisierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung des sauerstoffhaltigen Gases 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

In einer Ausführungsform wird in Gegenwart eines oder mehrerer Oxidationskatalysatoren gearbeitet, die selektiv die Verbrennung von Wasserstoff mit Sauerstoff zu Wasser in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der dehydrierende Aromatisierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Oxidationskatalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für den sauerstoffhaltige Gasstrom. Die Einspeisung von sauerstoffhaltigem Gasstrom und/oder wasserstoffhaltigem Gasstrom kann an einer oder mehreren Stellen des Reaktors erfolgen.

In einer speziellen Ausführungsform des erfindungsgemäßen Verfahrens erfolgt eine Zwischeneinspeisung von sauerstoffhaltigem Gasstrom und gegebenenfalls von wasserstoffhaltigem Gasstrom vor jeder Horde eines Hordenreaktors. In einer weiteren Ausführungsform des erfindungsgemäßen Verfahrens erfolgt die Einspeisung von sauerstoffhaltigem Gasstrom und gegebenenfalls von wasserstoffhaltigem Gasstrom vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem dehydrierenden Aromatisierungskatalysator. In einer weiteren Ausführungsform ist kein spezieller Oxidationskatalysator vorhanden.

Ein bevorzugter Oxidationskatalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Je nach Ausführungsform der autothermen dehydrierenden Aromatisierung von C₁-C₄-Alkanen kann der sauerstoffhaltige Gasstrom gemeinsam mit oder getrennt von dem Eduktstrom A in die Reaktionszone geleitet werden. Analoges gilt auch für den wasserstoffhaltigen Gasstrom.

Bei den Reaktoren handelt es sich in der Regel um Festbett- oder Fließbettreaktoren.

Die bei der Dehydrierung und Aromatisierung des C₁-C₄-Alkans, vorzugsweise des Methans, benötigte Temperatur kann durch Heizung erreicht werden. Es ist aber auch möglich einen Teil des Eduktstroms A, vorzugsweise des Methans, oder des unumgesetzten C₁-C₄-Alkans und/oder des gebildeten Wasserstoffes (der ggf. rückgeführt wurde) mit Sauerstoff so umzusetzen, dass die Reaktion autotherm durchgeführt werden kann. Dies kann beispielsweise nach dem Fachmann bekannten Methoden erfolgen, beispielsweise in einem Zweizonenreaktor. Hierbei wird in der ersten Zone der Sauerstoff umgesetzt und die freiwerdende Energie zu Erwärmung des Eduktstroms A verwendet; in der zweiten Zone findet dann die Dehydrierung und Aromatisierung statt. Es ist aber auch möglich die Umsetzung des Sauerstoffes unter Energiefreisetzung und die Dehydrierung und Aromatisierung parallel durchzuführen.

Die vorstehend genannte Umsetzung mit Sauerstoff kann in Form einer homogenen Gasphasenreaktion, einer Flamme, in einem Brenner oder an einem katalytischen Kontakt erfolgen.

Beispielsweise wird der kohlenwasserstoffhaltige Eduktstrom mit einem sauerstoffhaltigen Strom vereinigt und der Sauerstoff in einer Oxidationsreaktion umgesetzt.

Als Katalysatoren für die dehydrierende Aromatisierung unter Zugabe von Sauerstoff eignen sich insbesondere die von Claridge et al (Applied Catalysis, A: General: 89, 103 (1992)) beschriebenen Metalloxide, insbesondere Chlorid-promotiertes Mangan(IV)oxid. Die Reaktion wird demnach üblicherweise bei einer Temperatur von 800 bis 1100°C, vorzugsweise bei 900 bis 1100°C, in einem Druckbereich von 1 bis 25 bar, vorzugsweise 3 bis 20 bar, durchgeführt. Das molare Verhältnis von C₁-C₄-Alkan, insbesondere Methan, zu Sauerstoff liegt in der Regel bei 30:1 bis 5:1.

Weiterhin kann auch der von Anderson (Applied Catalysis 19, 141 (1985)) verwendete Katalysator H-ZSM-5 verwendet werden, insbesondere in Gegenwart von Stickoxid. Die Reaktion wird üblicherweise bei einer Temperatur von 250 bis 700°C, insbesondere bei 300 bis 600°C, in einem Druckbereich von 1 bis 10 bar, durchgeführt. Das molare Verhältnis von C₁-C₄-Alkan, insbesondere Methan, zu Stickoxid liegt in der Regel bei 80:20 bis 95:5.

In einer weiteren Ausgestaltungsform wird ohne Zufuhr von sauerstoffhaltigen Gasen gearbeitet.

Als Katalysatoren eignen sich insbesondere Zeolith-haltige, insbesondere ZSM-Zeolithe, wie beispielsweise ZSM-5, ZSM-8, ZSM-11, ZSM-23 und ZSM-35, vorzugsweise ZSM-5, oder MCM-Zeolithe, wie beispielsweise MCM-22. Die Katalysatoren können neben den Zeolithen ein oder mehrere Metalle aus den Gruppen IIIA, IB, IIB, VIB, VIIB und VIIIB enthalten.

Vorzugsweise werden Mo/HZSM-5-Katalysatoren verwendet, welche mit Cu, Co, Fe, Pt, Ru promotiert sein können. Es können aber auch W/HZSM-5, In/HZSM-5, Ga/HZSM-5, Zn/HZSM-5, Re/HZSM-5 eingesetzt werden, aber auch W/HZSM-5, promotiert mit Mn, Zn, Ga, Mo oder Co.

Ebenso vorzugsweise werden W/MCM-22-Katalysatoren verwendet, welche mit Zn, Ga, Co, Mo promotiert sein können. Es kann aber auch Re/HMCM-22 eingesetzt werden.

Insbesondere können Aluminosilicate vom Pentasil-Typ, verwendet werden, beispielsweise ZSM-5, ZSM-8, ZSM-11, ZSM-23 und ZSM-35, vorzugsweise ZSM-5. Diese können mit Molybdän, Zink, Gallium, vorzugsweise Molybdän modifiziert werden. Es ist auch möglich ein weiteres Metall zuzusetzen, beispielsweise Kupfer, aber auch Cobalt, Eisen, Platin oder Ruthenium. So werden beispielsweise Kupfer promotierte Mo/ZSM-5 Zeolith-Katalysatoren erhalten (Qi et al, Catalysis Today 98, 639 (2004)) oder mit einem Metall der Gruppe VIIIB des Periodensystems, insbesondere mit Rhenium, promotierte Ga/ZSM-5 Zeolith-Katalysatoren (US 4,727,206) erhalten. Besondere Molybdän-modifizierte ZSM-5 Zeolithe werden in WO 02/10099 beschrieben.

Weiterhin können auch MCM/22 Katalysatoren, welche mit W modifiziert und ggf. mit Zn, Ga, Co, Mo promotiert sind, eingesetzt werden. Ebenso eignet sich Re/HMCM-22.

Üblicherweise wird die dehydrierende Aromatisierung von C₁-C₄-Methan, welche ohne Zufuhr von sauerstoffhaltigen Gasströmen, betrieben wird, an den oben genannten Katalysatoren bei Temperaturen von 400 bis 1000 °C, bevorzugt von 500 bis 900°C, besonders bevorzugt von 600 bis 800°C, insbesondere von 700 bis 750 °C, bei einem Druck von 0.5 bis 100 bar, bevorzugt bei 1 bis 50 bar, besonders bevorzugt bei 1 bis 30 bar, insbesondere 1 bis 10 bar, durchgeführt. Üblicherweise wird die Umsetzung bei einer GHSV (Gas Hourly Space Velocity) von 100 bis 10 000 h⁻¹, vorzugsweise von 200 bis 3000 h⁻¹ durchgeführt.

Es kann von Vorteil sein, die, bei der dehydrierenden Aromatisierung von C₁-C₄-Alkanen, welche ohne Zufuhr von sauerstoffhaltigen Gasströmen betrieben wird, verwendeten Katalysatoren, vor der eigentlichen Verwendung zu aktivieren.

Diese Aktivierung kann mit einem C₂-C₄-Alkan, wie z.B. Ethan, Propan, Butan oder einem Gemisch hiervon, vorzugsweise Butan, erfolgen. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

Es ist aber auch möglich eine Aktivierung durchzuführen, indem der Eduktstrom A das C₂-C₄-Alkan, oder ein Gemisch hiervon, per se schon enthält oder das C₂-C₄-Alkan, oder ein Gemisch hiervon, dem Eduktstrom A zugesetzt wird. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

In einer weiteren Ausgestaltungsform ist es auch möglich zusätzlich zu dem C₂-C₄-Alkan noch Wasserstoff beizufügen.

Selbstverständlich können die, in diesem Verfahren, insbesondere wenn das Verfahren ohne Zusatz von sauerstoffhaltigen Gasströmen durchgeführt wird, eingesetzten Katalysatoren, bei nachlassender Aktivität nach üblichen, dem Fachmann bekannten Methoden regeneriert werden. Hier sei insbesondere die Behandlung mit einem sauerstoffhaltigen Gasstrom, wie z.B. Luft, angereicherte Luft oder Reinsauerstoff, aufgeführt, indem der sauerstoffhaltige Gasstrom anstelle des Eduktstromes A über den Katalysator geleitet wird. Es ist beispielsweise aber auch möglich, die Katalysatoren mit Wasserstoff zu regenerieren. Dies kann erfolgen indem man dem Eduktstrom A beispielsweise einen Wasserstoffstrom zusetzt. Das Verhältnis von Wasserstoffstrom zu Eduktstrom A liegt üblicherweise im Bereich von 1:1000 bis 2:1, bevorzugt 1:500 bis 1:5. Es kann sich aber auch anbieten abwechselnd Eduktstrom A und Wasserstoff über den Katalysator zu leiten.

Der Eduktstrom A wird in einer Reaktionszone mit dem Katalysator in Kontakt gebracht, wobei die Reaktionszone u.a. durch einen Reaktor, mehrere in Serie geschaltete Reaktoren oder eine bzw. mehrere kaskadierte Reaktoren, repräsentiert werden kann.

Die dehydrierende Aromatisierung kann grundsätzlich in allen aus dem Stand der Technik bekannten Reaktortypen durchgeführt werden. Eine vergleichsweise ausführliche Beschreibung von erfindungsgemäß geeigneten Reaktortypen enthält auch "Catalytica® Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator als Festbett in einem Reaktionsrohr oder in einem Bündel von Reaktionsrohren. Übliche Reaktionsrohr-Innendurchmesser betragen etwa 10 bis 15 cm. Ein typischer dehydrierender Aromatisierungsrohrbündelreaktor umfasst ca. 300 bis 1000 Reaktionsrohre. Die Katalysatorgeometrie kann beispielsweise kugelförmig oder zylindrisch (hohl oder voll), ring-, sattel- oder tablettenförmig sein. Weiterhin kommen Extrudate z. B. in Strang-, Trilobe-, Quadrulobe-, Stern- oder Hohlzylinderform in Frage.

Die dehydrierende Aromatisierung kann auch heterogen katalysiert im Wirbelbett durchgeführt werden. In einer besonderen Ausgestaltungsform enthält der Reaktor ein Wirbelbett, es kann aber auch zweckmäßig sein, mehrere Wirbelbetten nebeneinander zu betreiben, von denen sich einer oder mehrere in der Regel im Zustand der Regenerierung oder Reaktivierung befinden. Die für die dehydrierende Aromatisierung erforderliche Wärme kann dabei in das Reaktionssystem eingebracht werden, indem der Katalysator auf die Reaktionstemperatur vorerhitzt wird. Durch die Zumischung eines sauerstoffhaltigen Gasstromes kann auf die Vorerhitzer verzichtet werden, und die benötigte Wärme wird direkt im Reaktorsystem durch Verbrennung von Wasserstoff und/oder Kohlenwasserstoffen in Gegenwart von Sauerstoff erzeugt. (autotherme Fahrweise).

Die dehydrierende Aromatisierung kann in einem Hordenreaktor durchgeführt. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einem Hordenreaktor mit nur einer Horde. Die Durchführung der dehydrierenden Aromatisierung in einem einzelnen Schachtofenreaktor entspricht einer Ausführungsform. In einer weiteren bevorzugten Ausführungsform wird die dehydrierende Aromatisierung in einem Hordenreaktor mit 3 Katalysatorbetten durchgeführt.

Der Produktstrom B enthält vorzugsweise einen oder mehrere aromatische Kohlenwasserstoffe ausgewählt aus der Gruppe Benzol, Toluol, Ethylbenzol, Styrol, Xylol und Naphthalin. Insbesondere enthält der Produktstrom B als aromatischen Kohlenwasserstoff Benzol, Naphthalin oder Gemische hiervon, besonders bevorzugt Benzol, ebenso besonders bevorzugt Benzol und Naphthalin.

Die Ausbeute an aromatischen Kohlenwasserstoff(en) (bezogen auf umgesetztes Alkan aus dem Eduktstrom A) liegt dabei im Bereich von 1 bis 95 %, bevorzugt von 5 bis 80 %, vorzugsweise von 10 bis 60 %, besonders bevorzugt von 15 bis 40 %.

Die Selektivität an aromatischen Kohlenwasserstoff(en) (bezogen auf umgesetztes Alkan aus dem Eduktstrom A) beträgt dabei mindestens 10 %, bevorzugt 30 %, besonders bevorzugt 50 %, außerordentlich bevorzugt 70%, insbesondere 90 %.

Weiterhin enthält der Produktstrom B neben unumgesetzten C₁-C₄-Alkan, oder ein Gemisch aus unumgesetzten C₁-C₄-Alkanen, und gebildeten Wasserstoff, bereits im Eduktstrom A enthaltene Inerte wie Stickstoff, Helium (und ggf. Alkane wie Ethan, Propan etc.) sowie gebildete Nebenprodukte und andere bereits in Eduktstrom A enthaltene Verunreinigungen sowie ggf. (zum Teil) dem Eduktstrom A zugesetzte Gasströme.

Falls autotherm gearbeitet kann der der Produktstrom zusätzlich noch das bei der Umsetzung mit Sauerstoff gebildete Wasser, Kohlenmonoxid und/oder Kohlendioxid enthalten.

In einer speziellen Ausführungsform kann die partielle Rückführung des Produktstromes B, also vor der Abtrennung von Schwersiedern, durchgeführt werden. Hierzu wird ein Teil des aus der Reaktionszone kommenden Produktstromes B wieder in die Reaktionszone geführt. Dies kann dabei wahlweise durch die direkte Dosierung in die Reaktionszone oder durch die vorige Vereinigung mit dem Eduktstrom A erfolgen. Der Anteil des rückgeführten Stromes beträgt zwischen 1 und 95 % des Produktstromes B, bevorzugt zwischen 5 und 90 % des Produktstromes B.

Alternativ zu obiger Rückführung kann in einer weiteren Ausgestaltungsform auch eine Rückführung eines Teils der Leichtsiederstrome C bzw. C' erfolgen. Diese Leichtsiederströme C bzw. C' werden erhalten, indem man aus dem Produktstrom B partiell oder vollständig die Schwersieder bzw. die aromatischen Kohlenwasserstoffe abtrennt. Ein Teil der Ströme C und/oder C' werden dabei wahlweise durch die direkte Dosierung in die Reaktionszone oder durch die vorige Vereinigung mit dem Eduktstrom A zurückgeführt. Der Anteil des rückgeführten Stromes beträgt zwischen 1 und 95 % des entsprechenden Stromes C oder C', bevorzugt zwischen 5 und 90 % des entsprechenden Stromes C oder C'.

Ggf. können die rückgeführten Ströme ganz oder teilweise von Wasserstoff befreit werden.

Die Rückführung eines Stromes kann z.B. unter Zuhilfenahme eines Verdichters, eines Gebläses oder einer Düse erfolgen. In einer bevorzugten Ausführungsform ist die Düse eine Treibstrahldüse, wobei der Eduktstrom A oder einsauerstoffhaltiger Strom oder ein Dampfstrom als Treibmedium verwendet wird.

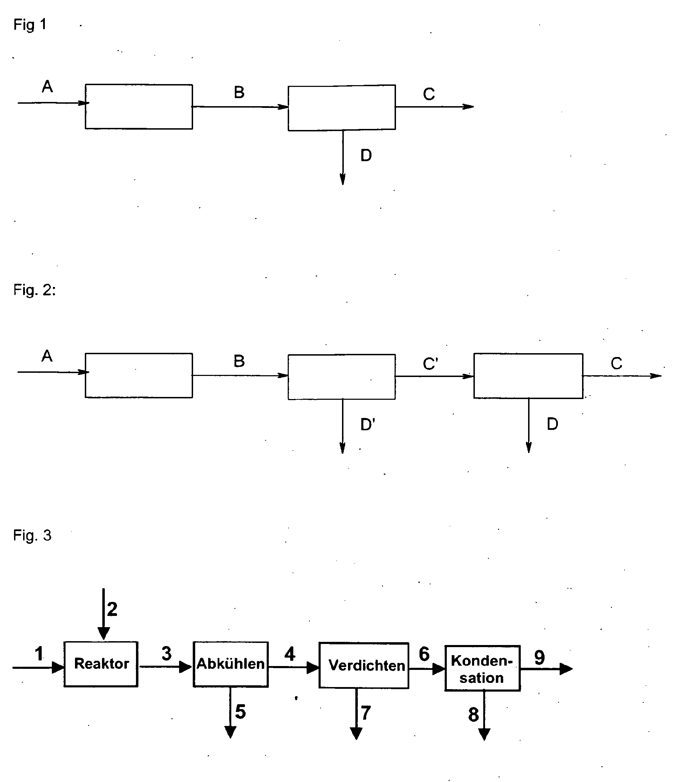

Die Trennung des Produktstromes B in den Leichtsiederstrom C und den Schwersiederstrom D erfolgt durch Kondensation oder auch fraktionierte Kondensation. Unter fraktionierter Kondensation wird hier eine mehrstufige Destillation im Beisein größerer Inertgasmengen verstanden. So kann der Produktstrom B auf-30°C bis 80°C, vorzugsweise auf 0°C bis 70°C, besonders bevorzugt auf 30°C bis 60°C abgekühlt werden. Hierbei kondensieren die aromatischen Kohlenwasserstoffe und Schwersieder während das nicht umgesetzte Methan und der gebildete Wasserstoff gasförmig vorliegen und somit nach üblichen Methoden abgetrennt werden können. Ggf. enthält der Leichtsiederstrom C auch die oben genanten Inerte und Alkane sowie die gebildeten Nebenprodukte und/oder bereits in Eduktstrom A enthaltene Verunreinigungen sowie ggf. (zum Teil) dem Eduktstrom A zugesetzte Gasströme (Fig. 1).

In einer besonderen Ausgestaltungsform, kann es von Vorteil sein, den Produktstrom B in mehreren Stufen von Schwersiedern zu befreien. Hierzu wird dieser beispielsweise auf -30°C bis 80°C abgekühlt, der Schwersiederstrom D', welcher einen Teil der Schwersieder enthält, abgetrennt und der Leichtsiederstrom C' verdichtet und weiter gekühlt, so dass man den Schwersiederstrom D und den Leichtsiederstrom C erhält. Eine Verdichtung erfolgt vorzugsweise auf ein Druckniveau von 5 bis 100 bar, bevorzugt 10 bis 75 bar und weiter bevorzugt 15 bis 50 bar. Um eine weitgehende Kondensation einer bestimmten Verbindung zu erreichen zu erreichen wird eine entsprechend angemessene Temperatur eingestellt. Erfolgt die Kondensation unterhalb 0°C ist ggf. eine vorherige Trocknung des Gases notwendig. (Fig. 2)

Der Schwersiederstrom D enthält vornehmlich die leichteren aromatischen Kohlenwasserstoffe, wie z.B. Benzol, und der Leichtsiederstrom C enthält die nicht umgesetzten C₁-C₄-Alkane, vorzugsweise Methan, den gebildete Wasserstoff und ggf. die oben genannten Inerte sowie die gebildeten leichtflüchtigen Nebenprodukte und/oder bereits in Eduktstrom A enthaltenen Verunreinigungen. Das unumgesetzte C₁-C₄-Alkan, vorzugsweise Methan, und der gebildete Wasserstoff können gewünschtenfalls nach üblichen Methoden getrennt werden.

Weiterhin kann der Leichtsiederstrom C bei autothermer Fahrweise das bei der Umsetzung mit Sauerstoff gebildete Kohlenmonoxid, Kohlendioxid enthalten.

Die im Schwersiederstrom D enthaltenen aromatischen Kohlenwasserstoffe können nach den üblichen Methoden getrennt und/oder aufgereinigt werden. Ggf. kann der Schwersiederstrom D bei autothermer Fahrweise das bei der Umsetzung mit Sauerstoff gebildete Wasser enthalten, das in üblicher Weise, z.B. über einen Phasenscheider, abgetrennt werden kann.

Je nach Fahrweise der dehydrierenden Aromatisierung (unter Zugabe von Sauerstoff oder ohne Zugabe von Sauerstoff) und den Anforderungen des weiterführenden Prozesses bzgl. der Reinheit, der Gegenwart störender Komponenten oder des Brennwertes des Leichtsiederstromes C kann die Abtrennung einzelner Nebenkomponenten vor der Weiterverwendung notwendig sein.

So kann beispielsweise bei der dehydrierenden Aromatisierung unter Zugabe von Sauerstoff unter Umständen Kohlenmonoxid oder Kohlendioxid gebildet werden. Bei der Verwendung von Luftsauerstoff können der mit in das System eingebrachte Stickstoff oder andere Inerte im Strom C auftreten:

Die dehydrierende Aromatisierung ist mit der Bildung von Wasserstoff verbunden, wodurch sich der Brennwert des Leichtsiederstromes C verändert.

In einer speziellen Ausgestaltungsform der vorliegenden Erfindung werden daher eine oder mehrerer dieser gebildeten Komponenten, die nicht C₁-C₄-Alkane sind, teilweise oder vollständig abgetrennt.

Hierzu wird beispielsweise in einem Verfahrensteil zwischen der Abtrennung der Schwersieder (D, D') und dem weiterführenden Prozess die nicht kondensierbaren oder leicht siedenden Gasbestandteile wie Wasserstoff, Sauerstoff, Kohlenmonoxid, Kohlendioxid, Stickstoff in einem Absorptions-/Desorptions-Zyklus mittels eines hoch siedenden Absorptionsmittels von den Kohlenwasserstoffen abgetrennt, wobei ein Strom erhalten wird, der die C₁-C₄-Kohlenwasserstoffe und das Absorptionsmittel enthält, und ein Abgasstrom, der die nicht kondensierbaren oder leicht siedenden Gasbestandteile enthält.

In der Absorptionsstufe eingesetzte inerte Absorptionsmittel sind im Allgemeinen hochsiedende unpolare Lösungsmittel, in denen das abzutrennende C₁-C₄-Kohlenwasserstoff-Gemisch eine deutlich höhere Löslichkeit als die übrigen abzutrennenden Gasbestandteile aufweist. Die Absorption kann durch einfaches Durchleiten des Stroms C durch das Absorptionsmittel erfolgen. Sie kann aber auch in Kolonnen erfolgen. Dabei kann im Gleichstrom, Gegenstrom oder Kreuzstrom gearbeitet werden. Geeignete Absorptionskolonnen sind z.B. Bodenkolonnen mit Glocken-, Ventil- und/oder Siebböden, Kolonnen mit strukturierten Packungen, z.B. Gewebepackungen oder Blechpackungen mit einer spezifischen Oberfläche von 100 bis 1000 m2/m3 wie Mellapak® 250 Y, und Füllkörperkolonnen, z.B. mit Kugeln, Ringen oder Sätteln aus Metall, Kunststoff oder Keramik als Füllkörpern. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher, Rotationswäscher sowie Blasensäulen mit und ohne Einbauten in Betracht.

Geeignete Absorptionsmittel sind vergleichsweise unpolare organische Lösungsmittel, beispielsweise aliphatische C₅-C₁₈-Alkene, Naphtha oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%. Geeignete Absorptionsmittel sind ferner Pentane, Hexane, Heptane, Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Alternativ kann auch gezielt Kohlendioxid mit einem selektiven Absorptionsmittel aus dem Strom C entfernt werden. Wiederum können in der Absorptionsstufe Absorptionsmittel, wie z.B. dem Fachmann bekannte basische Waschmittel, in denen das abzutrennende Kohlendioxid über eine deutlich höhere Löslichkeit als die übrigen abzutrennenden Gasbestandteile aufweist, eingesetzt werden. Die Absorption kann durch einfaches Durchleiten des Stroms C durch das Absorptionsmittel erfolgen. Sie kann aber auch in Kolonnen erfolgen. Dabei kann im Gleichstrom, Gegenstrom oder Kreuzstrom gearbeitet werden. Technisch kommen hierbei die zuvor aufgeführten apparativen Lösungen in Betracht.

Zur Abtrennung des im Abgasstrom enthaltenen Wasserstoffs kann dieser, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist.

Alternativ können einzelne Komponenten auch durch chemische Umsetzung abgetrennt werden. Durch Oxidation des entstandenen Wasserstoffs lässt dieser sich beispielsweise als Wasser aus dem Gemisch durch Kondensation entfernen.

Alternativ können Komponenten in einem Adsorptionsverfahren (thermische oder Druckwechsel-Adsorption) abgetrennt werden. Hierbei wird ein Adsorbens zyklisch in einer ersten Phase mit dem wasserstoffhaltigen Strom beaufschlagt, wobei alle Komponenten außer Wasserstoff, so auch die C₁-C₄-Alkane durch Adsorption zurückgehalten werden. In einer zweiten Phase werden diese Komponenten durch erniedrigten Druck oder erhöhte Temperatur wieder desorbiert.

Der so abgetrennte molekulare Wasserstoff kann bei Bedarf zumindest teilweise in einer Hydrierung eingesetzt oder aber einer sonstigen Verwertung zugeführt werden, beispielsweise zur Erzeugung elektrischer Energie in Brennstoffzellen. Alternativ kann der Abgasstrom verbrannt werden.

Alternativ kann bei genügend unterschiedlichen Siedepunkten auch die Rektifikation zur Abtrennung einzelner Komponenten genutzt werden.

Eine Abtrennung einzelner Komponenten ist im Allgemeinen nicht ganz vollständig, so dass in den C₁-C₄-Kohlenwasserstoffen - je nach Art der Abtrennung - noch geringe Mengen oder auch nur Spuren der weiteren Gasbestandteile vorliegen können.

Das im Leichtsiederstrom C enthaltene unumgesetzte C₁-C₄-Alkan und der gebildete Wasserstoff werden nun einem weiteren C₁-C₄-Alkan-verbrauchenden Verfahren, ausgewählt aus der Gruppe
i) Verbrennung in GUD-Kraftwerken;
ii) Darstellung von Synthesegas durch Dampf-Reformieren oder partieller Oxidation;
iii) Umsetzung mit Ammoniak zu Blausäure in Gegenwart von Sauerstoff nach dem Andrussow-Verfahren, oder ohne Sauerstoffzusatz nach dem BMA-Verfahren;
iv) Umsetzung mit Schwefel zu Schwefelkohlenstoff;
v) Pyrolyse zu Acetylen im Lichtboden oder nach dem Sachsse-Bartholome-Verfahren; und
vi) oxidative Kupplung zu Ethylen;
zugeführt.

Als C₂-C₄-Alkan-verbrauchende Prozesse seien i) und ii) genannt.

Es kann von Vorteil sein, den gebildeten Wasserstoff vor der Verwendung in dem Methan-verbrauchenden Prozess, wie beispielsweise den Prozessen ii) bis vi), partiell oder vollständig abzutrennen. Hierfür können die, dem Fachmann bekannte, oben aufgeführten Verfahren, eingesetzt werden und der so gewonnenen Wasserstoff seinerseits zur Energiegewinnung oder in einem Wasserstoffverbrauchenden Prozess, wie z.B. einer Hydrierung, eingesetzt werden. Es kann gegebenenfalls auch von Vorteil sein mit gängigen Verfahren die oben erwähnten Inerte, Alkane sowie die gebildete Nebenprodukte, und das bei autothermer Fahrweise gebildete Kohlendioxid, vor der Verwendung in dem Methan-verbrauchenden Prozeß abzutrennen.

Vorzugsweise wird der Leichtsiederstrom C der Verbrennung in Gas- und Dampfkraftwerke (GuD-Kraftwerke) zugeführt, die Wirkungsgrade von etwa 50 bis 60 % erreichen.

Ein GuD-Kraftwerk stellt eine Kraft-Wärme-Maschine dar, deren realer Wirkungsgrad über den Carnot'schen Wirkungsgrad, dem höchsten theoretisch möglichen Wirkungsgrad einer Wärmekraftmaschine, von der Temperaturdifferenz zwischen Wärmequelle und Temperatursenke abhängt. Die Temperaturquelle bei einem GuD-Kraftwerk entspricht dem Verbrennungsprozess, die Temperatursenke der Umgebungstemperatur oder dem Kühlwasser. Die theoretische Beziehung zwischen dem Wirkungsgrad ε einer Wärmekraftmaschine und der Temperaturdifferenz lautet: ε = 1-(T_{S}/T_{Q}), wobei T_{S} für die Temperatur der Temperatursenke und T_{Q} für die Temperatur der Wärmequelle steht, jeweils in K angegeben. Der Wirkungsgrad einer Wärmekraftmaschine ist demnach umso höher, je größer die Temperaturdifferenz zwischen T_{S} und T_{Q} ist. Für ein GuD-Kraftwerk bedeutet dies, dass bei gleichem Aufwand für die Kühlung (und somit gleicher Temperatur der Temperatursenke) der Wirkungsgrad umso höher ist, je höher die Temperatur des Verbrennungsprozesses ist.

Hierbei zeigt sich überraschenderweise neben den bereits vorstehend aufgeführten Vorteilen des erfindungsgemäßen Verfahrens ein weiterer Vorteil speziell dieser Ausführungsform des erfindungsgemäßen Verfahrens, bei der der Leichtsiederstrom C in Schritt c) des erfindungsgemäßen Verfahrens einem GuD-Kraftwerk zugeführt wird. Die mit dem Leichtsiederstrom C in dem GuD-Kraftwerk erreichten Verbrennungstemperaturen liegen signifikant über denen eines üblichen Methan-Brenngemischs (Erdgas), wie in Beispiel 3 zu sehen ist. Der Wirkungsgrad des GuD-Kraftwerks kann somit gesteigert werden und eine hohe Gesamteffizienz des Gesamtprozesses erreicht werden.

Neben der erhöhten Effizienz des Gesamtprozesses besitzt diese Ausführungsform der Erfindung einen weiteren Vorteil: ihre im Gesamtprozess günstige CO₂-Ökobilanz. Durch die Kohlenstoffverwertung in der ersten Stufe in einem chemischen Produkt ist das H:C-Verhältnis in dem aus der ersten Stufe resultierenden Gas (nach Abtrennung der Aromaten) höher. In der zweiten Prozessstufe muss also weniger Kohlenstoff verbrannt werden. Dieses Gas führt bei gleichem Brennwert somit zu geringeren CO₂-Emissionen.

In einer weiteren Ausführungsform der Erfindung wird der Leichtsiederstrom C in Stufe c) des erfindungsgemäßen Verfahrens als Synthesegas bei der Ammoniaksynthese eingesetzt. Im Vergleich zu dem normalerweise üblichen Einsatz von Erdgas zur Generierung des für die Ammoniaksynthese benötigten Wasserstoffs zeigt der erfindungsgemäße Einsatz des Leichtsiederstroms C zur Darstellung des zur Ammoniaksynthese nötigen Wasserstoffs einen signifikant niedrigeren Bedarf an Erdgas zur Heizung des Prozesses sowie einen deutlich geringeren Massestrom. Dies bedeutet, dass bei erfindungsgemäßem Einsatz des Leichtsiederstroms C zur Bildung von Synthesegas zur Ammoniakherstellung deutlich weniger Methan pro Tonne Ammoniak eingesetzt werden muss als dies bei Einsatz von Erdgas zur Synthesegasherstellung der Fall ist. Insbesondere bei Betrachtung des Gesamtprozesses zeigen sich die Vorteile dieser Ausführungsform. Es werden zwei kommerziell bedeutsame Produkte hergestellt, Aromaten und Ammoniak, wobei durch die Kombination der dehydrierenden Aromatisierung und Ammoniaksynthese der zweite Prozess unter vorteilhafteren Bedingungen durchgeführt werden kann als bei direktem Einsatz von C₁-C₄-Alkan enthaltenden Gases bei der Ammoniaksynthese.

Gemäß dem erfindungsgemäßen Verfahren werden eine Selektivität an aromatischen Kohlenwasserstoff(en) (bezogen auf umgesetztes Alkan aus dem Eduktstrom A) im Bereich von mindestens 10%, bevorzugt 30%, besonders bevorzugt 50 %, außerordentlich bevorzugt 70%, insbesondere 90% erreicht.

### Beispiel 1: Kopplung mit GuD-Kraftwerk (Fig. 3, Tabelle 1)

Nachfolgend wurde eine erfindungsgemäße Ausführungsform rechnerisch simuliert, wobei die Anlage auf 100 kt/a Benzol und 20 kt/a Naphthalin ausgelegt wurden. Die dehydrierende Aromatisierung verläuft mit einer Selektivität von 71% bzgl. Benzol, 14 % bzgl. Naphthalin und 15 % bzgl. CO/CO₂. Der Umsatz von Methan liegt bei 23 %. Methan wird von ca. 50 bar auf 1,2 bar entspannt. Nach einer Vorwärmung auf 500°C wird Methan (Strom 1) bei einem Druck von 1,2 bar dem Reaktor zugeführt. Weiterhin wird Sauerstoff (Strom 2) zugeführt zur in-situ Erzeugung der benötigten Reaktionswärme. Bei 750°C verlässt der Strom 3 (Produktstrom B) den Reaktor. Der Strom 3 (Produktstrom B) wird abgekühlt. Das gebildete Kondensat 5 (Schwersiederstrom D') enthält vorwiegend Naphthalin und Wasser aber auch kleine Mengen Benzol kann und kann entsprechend aufgearbeitet werden. Der Gasstrom 4 (Leichtsiederstrom C') wird mehrstufig auf 30 bar verdichtet. Bei den Zwischenkühlungen fällt weiteres Kondensat (Strom 7) an, das im Wesentlichen aus Wasser besteht. Anschließend wird der Strom 6, der eine Temperatur 138°C hat, partiell kondensiert. Benzol und wiederum Wasser werden bei einem Druck von 30 bar abgetrennt. Das nicht umgesetzte Methan sowie der gebildete Wasserstoff und leichtsiedende Nebenprodukte werden als Strom 9 dem Kraftwerk zugeführt.

**Tabelle 1:**

| Strom-Nr | 1 | 2 | 3 | 4 | 5 | 6 | 7 | 8 | 9 |
|---|---|---|---|---|---|---|---|---|---|
| Menge [kg/h] | 96974 | 17914 | 114887 | 108761 | 6126 | 99901 | 8860 | 13989 | 85912 |
| C₆H₁₀ | 0,0 | 0,0 | 2,27 | 0,1 | 40,81 | 0,0 | 1,2 | 0,0 | 0,0 |
| C₆H₆ | 0,0 | 0,0 | 11,25 | 11,88 | 0,06 | 12,92 | 0,17 | 88,00 | 0,7 |
| H₂O | 0,0 | 0,0 | 12,03 | 9,38 | 59,09 | 1,51 | 98,09 | 10,60 | 0,03 |
| CH₄ | 100 | 0,0 | 64,75 | 68,40 | 0,0 | 74,46 | 0,0 | 0,0 | 86,59 |
| CO | 0,0 | 0,0 | 1,72 | 1,82 | 0,0 | 1,98 | 0,0 | 0,0 | 2,3 |
| CO₂ | 0,0 | 0,0 | 5,4 | 5,71 | 0,04 | 6,16 | 0,54 | 1,4 | 6,94 |
| H₂ | 0,0 | 0,0 | 2,58 | 2,73 | 0,0 | 2,97 | 0,0 | 0,0 | 3,45 |
| O₂ | 0,0 | 100,0 | 0,0 | 0,0 | 0,0 | 0,0 | 0,0 | 0,0 | 0,0 |
| | | | | | | | | | |
| T [°C] | 500 | 100 | 750 | 50 | 50 | 138 | 55 | 1 | 1 |
| p [bar] | 1,5 | 1,5 | 1,5 | 1,4 | 1,4 | 30 | 12,3 | 30 | 30 |

Die Angaben in den Spalten 1 bis 9 bzgl. C₆H₁₀, C₆H₆, H₂O, CO, CO₂, H₂ und O₂ sind Gew.-%-Angaben.

Dieses Beispiel zeigt deutlich, dass bei dem erfindungsgemäßen Verfahren, durch den Betrieb im geraden Durchgang bei der vorgegebenen Raumgeschwindigkeit entsprechende Umsätze erreicht werden können, und die Volumina der benötigten Apparaturen entsprechend kleiner dimensioniert werden können und die Stoffströme entsprechend geringer anfallen. Daher sind sowohl die Anschaffungskosten wie auch die Betriebskosten bei dem erfindungsgemäßen Verfahren niedrig.

### Beispiel 2

Als Katalysator kam ein Mo-/H-ZSM-5 Katalysator (3 Gew.-% Mo, Si:AI-Verhältnis von ca. 50 mol/mol) zum Einsatz. Dieser wurde in einer einstufigen Tränkung mittels einer wässrigen Lösung von Ammoniumheptamolybdat imprägniert, getrocknet und bei 500° C kalziniert.

Ca. 1 g des pulverförmigen Katalysators wurde unter Helium auf 500° C erwärmt. Bei dieser Temperatur wurde Methan zugesetzt und der Katalysator unter Helium/Methan (ca. 15 % Helium in Methan) stufenweise auf 750° C erwärmt. Dann wurde bei dieser Temperatur gegen äußere Atmosphäre (ca. 1 bar, Druckverlust über von ca. 1 bar) bei einer GHSV von ca. 1000 h⁻¹ die dehydrierende Aromatisierung untersucht (Tabelle 2).

**Tabelle 2:**

| Methan [Vol%] | Helium [Vol%] | O₂ [Vol%] | CO₂ [Vol%] | CO [Vol%] | H₂O [Vol%] | H₂ [Vol%] | Ethylen [Vol%] | Benzol [Vol%] | Toluol [Vol%] | Naphthalin. [Vol%] |
|---|---|---|---|---|---|---|---|---|---|---|
| 60,87 | 16,69 | 0,00 | 0,00 | 0,15 | 0,00 | 11,15 | 0,00 | 1,21 | 0,05 | 0,04 |

### Beispiel 3:

Nachfolgend wurde die Verbrennung eines üblicherweise in GuD-Kraftwerken verwendeten C₁-C₄-Alkan enthaltenden Gasgemischs (a) (Erdgas, nicht erfindungsgemäß) sowie die Verbrennung einer Leichtsiederfraktion C (b) (erfindungsgemäß, Abgas DHAM) theoretisch berechnet.
a) C₁-C₄-Alkane enthaltendes Gasgemisch (Erdgas; nicht erfindungsgemäß)

**Tabelle 3**

| Zusammensetzung | |
|---|---|
| H₂ | 0,00% |
| CH₄ | 93,00% |
| C₂H₄ | 0,00% |
| C₂H₆ | 3,00% |
| C₃H₈ | 1,30% |
| C₄H₁₀ | 0,60% |
| CO₂ | 1,00% |
| N₂ | 1,10% |
| spezifische CO₂-Bildung: | 28,5 m³CO₂/kJ |
| theor. Verbrenungs temperatur: | 1944 °C |

b) Leichtsiederfraktion C (Abgas DHAM; erfindungsgemäß)

**Tabelle 4**

| Zusammensetzung | |
|---|---|
| H₂ | 30,00% |
| CH₄ | 64,00% |
| C₂H₄ | 1,00% |
| C₂H₆ | 2,00% |
| C₃H₈ | 1,30% |
| C₄H₁₀ | 0,60% |
| CO₂ | 0,00% |
| N₂ | 1,10% |

| | |
|---|---|
| spezifische CO₂-Bildung: | 25,4 m³CO₂/kJ |
| theor. Verbrennungstemperatur: | 1966 °C |

### Beispiel 4

Nachfolgend wurde der Einsatz von Methan (nicht erfindungsgemäß) sowie des Leichtsiederstroms C in der Ammoniaksynthese simuliert. Die Rechnungen sind für eine Ammoniakausbeute von 100 t/h durchgeführt worden.

In Tabelle 5 sind die jeweiligen Feed-Zusammensetzungen sowie die sich aus den Simulationsrechnungen ergebenden Stoffströme dargestellt.

**Tabelle 5**

| Feed | Methan (nicht erfindungsgemäß) | Leichtsiederstrom C (erfindungsgemäß) |
|---|---|---|
| Zusammensetzung | 100 % CH₄ | 22,6% H₂ + 77,4% CH₄ |
| H₂-Bedarf in kmol/h | 8824 | 8824 |
| erforderlicher Feed in kmol/h | 4412 | 4974 |
| erforderlicher Feed in t/h | 70,6 | 63,8 |

Das Verhältnis der Volumenströme bei Berücksichtigung realer Bedingungen beträgt: Feed (Leichtsiederstrom C)/Feed (Methan) = 1,20, das Verhältnis der Masseströme Feed (Leichtsiederstrom C)/Feed (Methan) = 0,94. Dies bedeutet, dass bei Einsatz des Leichtsiederstroms C ein um 20% höherer Volumenstrom, aber ein um 6% niedrigerer Massestrom und damit eine geringere Menge Methan nötig ist als bei Einsatz von reinem Methan. Das Verhältnis des Bedarfs an Erdgas zum Heizen des Prozesses beträgt: Heizerdgasbedarf (Leichtsiederstrom C)/Heizerdgasbedarf (Methan) = 0,942. Der Wärmebedarf bei der Synthesegasherstellung ist also bei erfindungsgemäßem Einsatz des Leichtsiederstroms C in der Ammoniaksynthese signifikant geringer als bei Verwendung von Methan als Edukt.

## Patentansprüche

1. Verfahren zur Herstellung von aromatischen Kohlenwasserstoffen aus einem Ci-C₄-Alkan, oder einem Gemisch von C₁-C₄-Alkanen, **dadurch gekennzeichnet, dass** man
a) einen Eduktstrom A, der ein C₁-C₄-Alkan, oder ein Gemisch von C₁-C₄-Alkanen, enthält, wobei der Eduktstrom A mindestens 70 mol-% Methan enthält, mit einem Katalysator in Kontakt bringt, und einen Teil des C₁-C₄-Alkans, oder einen Teil des Gemisches der C₁-C₄-Alkane, zu aromatischen Kohlenwasserstoff(en), umsetzt;
b) den aus Schritt a) resultierenden Produktstrom B, in einen Leichtsieder-strom C, welcher den Hauptteil des Wasserstoffs und des unumgesetzten C₁-C₄-Alkans, oder des Gemisches von C₁-C₄-Alkanen, enthält, und einen Schwersiederstrom D oder mehrere Schwersiederströme D', welcher oder welche den Hauptteil des gebildeten aromatischen Kohlenwasserstoffes enthält oder enthalten, auftrennt; und
c) den Leichtsiederstrom C einem weiteren C₁-C₄-Alkan-verbrauchenden Verfahren, ausgewählt aus der Gruppe
i) Verbrennung in einem Gas- und Dampf-Kraftwerk;
ii) Darstellung von Synthesegas durch Dampf-Reformieren oder partieller Oxidation;
iii) Umsetzung mit Ammoniak zu Blausäure in Gegenwart von Sauerstoff nach dem Andrussow-Verfahren, oder ohne Sauerstoffzusatz nach dem BMA-Verfahren;
iv) Umsetzung mit Schwefel zu Schwefelkohlenstoff;
v) Pyrolyse zu Acetylen im Lichtboden oder nach dem Sachsse-Bartholome-Verfahren; und
vi) oxidative Kupplung zu Ethylen;
zuführt.

2. Verfahren gemäß Anspruch 1, wobei zu dem Eduktstrom A Wasserdampf, Wasserstoff, Kohlenmonoxid, Kohlendioxid, ein oder mehrere Edelgase, und/oder sauerstoffhaltige Gasströme, zugesetzt wird.

3. Verfahren nach den Ansprüchen 1 oder 2, wobei die dehydrierende Aromatisierung des C₁-C₄-Alkans, oder eines Gemisches von C₁-C₄-Alkanen, unter Zufuhr von sauerstoffhaltigen Gasströmen, erfolgt.

4. Verfahren nach den Ansprüchen 1 oder 2, wobei die dehydrierende Aromatisierung des C₁-C₄-Alkans, oder eines Gemisches von C₁-C₄-Alkanen, ohne Zufuhr von sauerstoffhaltigen Gasströmen, erfolgt.

5. Verfahren nach den Ansprüchen 1 bis 4, wobei die dehydrierende Aromatisierung des C₁-C₄-Alkans, oder eines Gemisches von C₁-C₄-Alkanen, in Gegenwart eines Zeolith-haltigen Katalysators durchgeführt wird.

6. Verfahren nach Anspruch 5, wobei der Katalysator durch Behandlung mit einem C₂-C₄-Alkan, oder eines Gemisches hiervon, aktiviert wird.

7. Verfahren nach den Ansprüchen 1 bis 6, wobei die dehydrierende Aromatisierung des C₁-C₄-Alkans, oder eines Gemisches von C₁-C₄-Alkanen, bei einer Temperatur von 400 bis 1000°C, bevorzugt von 500 bis 900°C, besonders bevorzugt von 600 bis 800°C, insbesondere von 700 bis 750°C und einem Gesamtdruck von 0,5 bis 100 bar, bevorzugt von 1 bis 50 bar, besonders bevorzugt von 1 bis 30 bar, insbesondere von 1 bis 10 bar, außerordentlich bevorzugt von 1 bis 5 bar durchgeführt wird.

8. Verfahren nach den Ansprüchen 1 bis 7, **dadurch gekennzeichnet, dass** das Verfahren autotherm betrieben wird.

9. Verfahren nach den Ansprüchen 1 bis 8, **dadurch gekennzeichnet, dass** man einen Teil des Produktstromes B vor Abtrennung der Schwersieder bzw. aromatische Kohlenwasserstoffe in die Reaktionszone zurückführt.

10. Verfahren nach den Ansprüchen 1 bis 8, **dadurch gekennzeichnet, dass** man einen Teil des Produktstromes B nach partieller oder vollständiger Abtrennung von Hochsiedem bzw. aromatische Kohlenwasserstoffe in die Reaktionszone zurückführt

11. Verfahren nach den Ansprüchen 1 bis 10, wobei der im Produktstrom B enthaltene aromatische Kohlenwasserstoff Benzol, Toluol, Ethylbenzol, Styrol, Xylol, Naphthalin, oder ein Gemisch hiervon, ist.

12. Verfahren nach Anspruch 11, wobei der im Produktstrom B enthaltene aromatische Kohlenwasserstoff Benzol ist.

13. Verfahren nach Anspruch 11, wobei die im Produktstrom B enthaltenen aromatischen Kohlenwasserstoffe Benzol und Naphthalin sind.

14. Verfahren nach einem der Ansprüche 1 bis 13, wobei das C₁-C₄-verbrauchende Verfahren die Verbrennung in einem Gas- und Dampf-Kraftwerk ist.

15. Verfahren nach den Ansprüchen 1 bis 14, **dadurch gekennzeichnet, dass** der Leichtsiederstrom C vor seiner Verwendung in einem weiterführenden Verfahren von einer oder mehrerer Komponenten, bei denen es sich nicht um ein C₁-C₄-Alkan, oder ein Gemisch von C₁-C₄-Alkanen, handelt, teilweise oder vollständig in einer Stofftrennung befreit wird.

16. Verfahren nach Anspruch 15, **dadurch gekennzeichnet, dass** es sich bei der Stofftrennung um einen Adsorptions-, Absorptions-, Membran- oder Rektifikationstrennungsschritt oder eine Abtrennung durch chemische Umsetzung handelt.

17. Verfahren nach den Ansprüchen 1 bis 16, wobei man den im Leichtsiederstrom C enthaltenen Wasserstoff partiell oder vollständig abtrennt.

18. Verfahren nach den Ansprüchen 1 bis 17, **dadurch gekennzeichnet, dass** das C₁-C₄-verbrauchende Verfahren in Schritt c) die Darstellung von Synthesegas für die Ammoniaksynthese ist.

19. Verfahren nach Anspruch 18, bei dem sich an die Darstellung von Synthesegas aus dem Leichtsiederstrom C die Ammoniaksynthese direkt anschließt.

## Claims

1. A method for producing an aromatic hydrocarbon from a C₁-C₄-alkane, or a mixture of C₁-C₄-alkanes, which comprises
a) bringing a feedstock stream A which comprises a C₁-C₄-alkane, or a mixture of C₁-C₄-alkanes, the feedstock stream A comprising at least 70 mol% methane, into contact with a catalyst and reacting a part of the C₁-C₄-alkane, or a part of the mixture of the C₁-C₄-alkanes, to form aromatic hydrocarbon(s);
b) fractionating the product stream B resulting from step a) into a low-boiler stream C which comprises the majority of the hydrogen and of the unreacted C₁-C₄-alkane, or of the mixture of C₁-C₄-alkanes, and a high-boiler stream D, or a plurality of high-boiler streams D', which stream or streams comprises or comprise the majority of the aromatic hydrocarbon formed; and
c) feeding the low-boiler stream C to a further C₁-C₄-alkane-consuming method, selected from the group
i) combustion in a combined-cycle power station;
ii) making synthesis gas via steam reformers or partial oxidation;
iii) reaction with ammonia to give prussic acid in the presence of oxygen by the Andrussow method, or without addition of oxygen by the BMA method;
iv) reaction with sulfur to give carbon disulfide;
v) pyrolysis to give acetylene in electric arc, or by the Sachsse-Bartholome method; and
vi) oxidative coupling to give ethylene.

2. The method according to claim 1, steam, hydrogen, carbon monoxide, carbon dioxide, one or more noble gases and/or oxygen-comprising gas streams being added to the feedstock stream A.

3. The method according to claims 1 and 2, the dehydrogenating aromatization of the C₁-C₄-alkane, or of a mixture of C₁-C₄-alkanes, proceeding with feed of oxygen-comprising gas streams.

4. The method according to claims 1 and 2, the dehydrogenating aromatization of the C₁-C₄-alkane, or of the mixture of C₁-C₄-alkanes, proceeding without feed of oxygen-comprising gas streams.

5. The method according to claims 1 to 4, the dehydrogenating aromatization of the C₁-C₄-alkane, or of a mixture of C₁-C₄-alkanes, being carried out in the presence of a zeolite-comprising catalyst.

6. The method according to claim 5, the catalyst being activated by treatment with a C₂-C₄-alkane, or a mixture thereof.

7. The method according to claims 1 to 6, the dehydrogenating aromatization of the C₁-C₄-alkane, or of a mixture of C₁-C₄-alkanes, being carried out at a temperature from 400 to 1000°C, preferably from 500 to 900°C, particularly preferably from 600 to 800°C, in particular from 700 to 750°C, and at a total pressure from 0.5 to 100 bar, preferably from 1 to 50 bar, particularly preferably from 1 to 30 bar, in particular from 1 to 10 bar, exceptionally preferably from 1 to 5 bar.

8. The method according to claims 1 to 7, which comprises carrying out the method autothermally.

9. The method according to claims 1 to 8, which comprises recirculating to the reaction zone a part of the product stream B before separating off the high boilers or aromatic hydrocarbons.

10. The method according to claims 1 to 8, which comprises recirculating to the reaction zone a part of the product stream B after partially or completely separating off high boilers or aromatic hydrocarbons.

11. The method according to claims 1 to 10, the aromatic hydrocarbon present in the product stream B being benzene, toluene, ethylbenzene, styrene, xylene, naphthalene, or a mixture thereof.

12. The method according to claim 11, the aromatic hydrocarbon present in the product stream B being benzene.

13. The method according to claim 11, the aromatic hydrocarbons present in the product stream B being benzene and naphthalene.

14. The method according to any of claims 1 to 13, the C₁-C₄-consuming method being combustion in a combined-cycle power station.

15. The method according to claims 1 to 14, wherein the low-boiler stream C, before its use in a continuing method, is partially or completely freed in a separation from one or more components which are not C₁-C₄-alkane, or a mixture of C₁-C₄-alkanes.

16. The method according to claim 15, wherein the separation is an adsorption, absorption, membrane or rectification separation step, or separation by chemical reaction.

17. The method according to claims 1 to 16, the hydrogen present in the low-boiler stream C being partially or completely separated off.

18. The method according to claims 1 to 17, wherein the C₁-C₄-consuming method in step c) is making synthesis gas for the ammonia synthesis.

19. The method according to claim 18, the ammonia synthesis following directly from the making of synthesis gas from the low-boiler stream C.

## Revendications

1. Procédé de fabrication d'hydrocarbures aromatiques à partir d'un alcane en C₁-C₄ ou d'un mélange d'alcanes en C₁-C₄, **caractérisé en ce que**
a) un courant de réactifs A, qui contient un alcane en C₁-C₄ ou un mélange d'alcanes en C₁-C₄, le courant de réactifs A contenant au moins 70 % en moles de méthane, est mis en contact avec un catalyseur et une partie de l'alcane en C₁-C₄ ou une partie du mélange des alcanes en C₁-C₄ est transformée en un ou plusieurs hydrocarbures aromatiques ;
b) le courant de produits B résultant de l'étape a) est séparé en un courant de composés de point d'ébullition faible C, qui contient la majeure partie de l'hydrogène et de l'alcane en C₁-C₄ ou du mélange d'alcanes en C₁-C₄ non réagi, et un courant de composés de point d'ébullition élevé D ou plusieurs courants de composés de point d'ébullition élevé D', qui contient ou qui contiennent la majeure partie de l'hydrocarbure aromatique formé ; et
c) le courant de composés de point d'ébullition faible C est introduit dans un autre procédé utilisant l'alcane en C₁-C₄, choisi dans le groupe constitué par :
i) une combustion dans une centrale à gaz et à vapeur ;
ii) une préparation de gaz de synthèse par reformage à la vapeur ou oxydation partielle ;
iii) une réaction avec de l'ammoniac en acide cyanhydrique en présence d'oxygène selon le procédé Andrussow ou sans ajout d'oxygène selon le procédé BMA ;
iv) une réaction avec du soufre en carbure de soufre ;
v) une pyrolyse en acétylène dans un arc électrique ou selon le procédé Sachsse-Bartholomé ; et
vi) un couplage oxydatif en éthylène.

2. Procédé selon la revendication 1, dans lequel de la vapeur d'eau, de l'hydrogène, du monoxyde de carbone, du dioxyde de carbone, un ou plusieurs gaz nobles et/ou des courants gazeux contenant de l'oxygène sont ajoutés au courant de réactifs A.

3. Procédé selon les revendications 1 ou 2, dans lequel l'aromatisation déshydrogénante de l'alcane en C₁-C₄ ou d'un mélange d'alcanes en C₁-C₄ a lieu avec introduction de courants gazeux contenant de l'oxygène.

4. Procédé selon les revendications 1 ou 2, dans lequel l'aromatisation déshydrogénante de l'alcane en C₁-C₄ ou d'un mélange d'alcanes en C₁-C₄ a lieu sans introduction de courants gazeux contenant de l'oxygène.

5. Procédé selon les revendications 1 à 4, dans lequel l'aromatisation déshydrogénante de l'alcane en C₁-C₄ ou d'un mélange d'alcanes en C₁-C₄ est réalisée en présence d'un catalyseur contenant une zéolithe.

6. Procédé selon la revendication 5, dans lequel le catalyseur est activé par traitement avec un alcane en C₂-C₄ ou un mélange de celui-ci.

7. Procédé selon les revendications 1 à 6, dans lequel l'aromatisation déshydrogénante de l'alcane en C₁-C₄ ou d'un mélange d'alcanes en C₁-C₄ est réalisée à une température de 400 à 1 000 °C, de préférence de 500 à 900 °C, de manière particulièrement préférée de 600 à 800 °C, notamment de 700 à 750 °C, et à une pression totale de 0,5 à 100 bar, de préférence de 1 à 50 bar, de manière particulièrement préférée de 1 à 30 bar, notamment de 1 à 10 bar, de manière exceptionnellement préférée de 1 à 5 bar.

8. Procédé selon les revendications 1 à 7, **caractérisé en ce que** le procédé est réalisé de manière autotherme.

9. Procédé selon les revendications 1 à 8, **caractérisé en ce qu'**une partie du courant de produits B est recyclée dans la zone de réaction avant la séparation des composés de point d'ébullition élevé ou des hydrocarbures aromatiques.

10. Procédé selon les revendications 1 à 8, **caractérisé en ce qu'**une partie du courant de produits B est recyclée dans la zone de réaction après une séparation partielle ou totale des composés de point d'ébullition élevé ou des hydrocarbures aromatiques.

11. Procédé selon les revendications 1 à 10, dans lequel l'hydrocarbure aromatique contenu dans le courant de produits B est le benzène, le toluène, l'éthylbenzène, le styrène, le xylène, la naphtaline ou un de leurs mélanges.

12. Procédé selon la revendication 11, dans lequel l'hydrocarbure aromatique contenu dans le courant de produits B est le benzène.

13. Procédé selon la revendication 11, dans lequel les hydrocarbures aromatiques contenus dans le courant de produits B sont le benzène et la naphtaline.

14. Procédé selon l'une quelconque des revendications 1 à 13, dans lequel le procédé utilisant un composé en C₁-C₄ est la combustion dans une centrale à gaz et à vapeur.

15. Procédé selon les revendications 1 à 14, **caractérisé en ce que** le courant de composés de point d'ébullition faible C est débarrassé en partie ou en totalité d'un ou de plusieurs composants, qui ne sont pas un alcane en C₁-C₄ ou un mélange d'alcanes en C₁-C₄, dans une séparation de matière avant son utilisation dans un procédé ultérieur.

16. Procédé selon la revendication 15, **caractérisé en ce que** la séparation de matière est une étape de séparation par adsorption, par absorption, sur membrane ou par rectification ou une séparation par réaction chimique.

17. Procédé selon les revendications 1 à 16, dans lequel l'hydrogène contenu dans le courant de composés de point d'ébullition faible C est séparé en partie ou en totalité.

18. Procédé selon les revendications 1 à 17, **caractérisé en ce que** le procédé utilisant un composé en C₁-C₄ à l'étape c) est la préparation d'un gaz de synthèse pour la synthèse d'ammoniac.

19. Procédé selon la revendication 18, dans lequel la synthèse d'ammoniac suit directement la préparation d'un gaz de synthèse à partir du courant de composés de point d'ébullition faible C.
